# FASCICULE DE BREVET EUROPEEN

(11) **EP 0 741 183 B1**
(45) Date de publication et mention de la délivrance du brevet: **24.03.1999**
(21) Numéro de dépôt: 95201151.8
(22) Date de dépôt: 04.05.1995
(51) Int. Cl.: C11C 1/00, C07C 51/48, C07C 57/12

(54) **Procédé de fractionnement d'acides gras**
Verfahren zur Fraktionierung von Fettsäuren
Process for the fractionation of fatty acids

(43) Date de publication de la demande: 06.11.1996
(73) Titulaire: SOCIETE DES PRODUITS NESTLE S.A., 1800 Vevey (CH)
(72) Inventeur: Sandoz, Laurence, CH-1077 Servion (CH); Wille, Hans-Juergen, CH-1844 Villeneuve (CH)
(74) Mandataire: Archambault, Jean

(56) Documents cités:
- EP-A- 0 421 867
- JOURNAL OF THE AMERICAN OIL CHEMISTS' SOCIETY, vol. 71, no. 6, 1994 CHAMPAIGN US, pages 563-567, M.S.K. SYED RAHMATULLAH ET AL. 'Gamma-linolenic acid concentrates from borage and evening primrose oil fatty acids via lipase-catalyzed esterification'
- JOURNAL OF THE AMERICAN OIL CHEMISTS' SOCIETY, vol. 71, no. 6, 1994 CHAMPAIGN US, pages 569-573, M.S.K. SYED RAHMATULLAH ET AL. 'Enrichment of gamma-linolenic acid from evening primrose oil and borage oil via lipase-catalyzed hydrolysis'
- DATABASE WPI Week 9106 Derwent Publications Ltd., London, GB; AN 91-041136 & JP-A-02 308 896 (SHOKUHIN SANGYO BIOREACTOR) , 21 Décembre 1990
- DATABASE WPI Week 8939 Derwent Publications Ltd., London, GB; AN 89-282553 & JP-A-01 207 258 (SHOKUHIN SANGYO BIOREACTOR) , 21 Août 1989
- DATABASE WPI Week 8422 Derwent Publications Ltd., London, GB; AN 84-137484 & JP-A-59 071 397 (NIPPON OILS & FATS KK) , 23 Avril 1984

## Description

L'invention concerne un procédé essentiellement enzymatique de fractionnement d'acides gras polyinsaturés.

Les acides gras des séries n-6 et n-3 présentent un intérêt nutritionnel, en particulier en tant que précurseurs de la biosynthèse des prostaglandines. Il peut être avantageux de disposer de fractions enrichies en ces acides gras pour différentes applications nutritionnelles et cosmétiques. On trouve ces acides gras dans la nature principalement sous forme de triglycérides. On obtient industriellement les acides gras libres à partir des triglycérides par hydrolyse à haute température et sous forte pression. Pour fractionner ensuite ces acides gras, on a développé plusieurs méthodes, par exemple la cristallisation, la distillation, la formation de complexes d'inclusion ou les techniques chromatographiques. L'application de ces méthodes peut entraîner une dégradation dans le cas des acides gras polyinsaturés ou s'avérer trop coûteuses pour pouvoir être appliquées industriellement.

Les méthodes enzymatiques représentent une alternative aux méthodes précédentes puisqu'elles permettent d'effectuer les réactions dans des conditions douces en utilisant peu d'énergie et un équipement moins sollicité.

On connaît, par exemple de Matthew J.Hills et coll. dans JAOCS, Vol 67, no. 9, p. 561-563, des procédés enzymatiques de fractionnement des acides gras de l'huile de poisson et de l'huile d'onagre reposant sur le fait que la cinétique de l'estérification par le butanol catalysée par les lipases de colza et de mucor miehei serait une fonction du degré de saturation de l'acide à estérifier. Après estérification des acides gras à plus grand degré de saturation, les esters sont séparés des acides gras n'ayant pas réagi par chromatographie en couche mince.

JAOCS, vol. 71, p. 563, col. 2 à p.564, col. 1, se rapporte à l'enrichissement enzymatique d'un mélange d'acides gras de l'huile de bourrache et de l'huile d'onagre par estérification des acides gras avec le n-butanol catalysée par une lipase, puis séparation des acides gras libres n'ayant pas réagi des esters butyliques par réaction du milieu additionné d'hexane avec la soude concentrée en milieu hydroéthanolique, séparation des phases organique et aqueuse. Le milieu aqueux est ensuite extrait par l'hexane, la phase hexane est de nouveau traitée par la soude en solution hydroéthanolique, d'une part les milieux hexane sont combinés et d'autre part les milieux aqueux sont réunis. On acidifie alors la phase aqueuse avec une solution concentrée d'acide chlorhydrique et on en extrait les acides gras libérés avec l'éther JAOCS, vol. 71, p. 569-573, en particulier p. 569, col. 2, alinéa 2-4, des mêmes auteurs, a trait à l'enrichissement en l'acide gamma-linolénique d'huile de bourrache ou d'onagre par hydrolyse enzymatique des triglycérides, puis séparation des acides gras libres non estérifiés par dissolution du produit de la lipolyse dans l'hexane et réaction des acides gras avec la soude concentrée en solution hydroéthanolique. La suite des opérations effectuées pour recueillir le mélange d'acides gras enrichi, par exemple en acide gamma-linolénique est analogue à ce qui est décrit dans l'article précédent ci-dessus.

Le but de l'invention est de mettre à disposition un procédé de fractionnement des acides gras d'huiles riches en acides gras polyinsaturés par estérification enzymatique qui soit applicable industriellement, en évitant une séparation chromatographique.

L'invention concerne donc un procédé essentiellement enzymatique de fractionnement d'acides gras polyinsaturés, caractérisé par le fait que l'on conduit l'estérification d'un mélange d'acides gras par catalyse enzymatique avec le méthanol, puis que l'on sépare les esters méthyliques formés des acides gras n'ayant pas réagi par saponification des acides gras par une base faible en milieu aqueux, que l'on extrait les savons formés à l'eau, que l'on acidifie la phase aqueuse, que l'on extrait les acides gras formés par l'hexane et que l'on obtient une fraction enrichie en acides gras polyinsaturés désirés.

Pour mettre en oeuvre le procédé selon l'invention, on utilise un mélange d'acides gras de départ provenant, de préférence, de l'hydrolyse enzymatique totale des triglycérides d'une huile riche en acides gras polyinsaturés. Cette hydrolyse a lieu de préférence en milieu émulsionné par une lipase non régio-spécifique de Candida cylindracea, de préférence à la température ambiante, à pH neutre ou proche de la neutralité et à la pression atmosphérique.

On peut citer comme huile de départ, toute huile naturelle d'origine animale ou végétale ou synthétique contenant des acides gras polyinsaturés, notamment de degré d'insaturation 3 et plus, par exemple l'huile de pépins de cassis, l'huile de bourrache, l'huile d'onagre, riches en acide gammalinolénique (GLA) ou l'huile "TGA" riche en acide arachidonique.

L'estérification a lieu en présence de méthanol et de solvant, par exemple d'hexane ou d'une faible quantité d'eau. La réaction dure 5 à 70 h et de préférence 10 à 40 h, de préférence à la température ambiante. L'enzyme utilisée peut être immobilisée ou non. Elle est de préférence immobilisée pour pouvoir être réutilisée. Elle peut être régio-spécifique ou non.

Après réaction, on obtient un mélange d'acides gras libres et d'esters méthyliques d'acides gras. La fraction estérifiée est appauvrie en acides gras de haut degré d'insaturation, par exemple dans le cas de l'huile de pépins de cassis particulièrement en GLA et en acide stéaridonique (SA), ce qui indique que ces acides ne sont presque pas estérifiés et par conséquent enrichis dans la fraction des acides gras libres.

Selon l'invention, la séparation des acides gras libres d'avec les esters a lieu par saponification des acides gras dans des conditions douces. Ces conditions se caractérisent par une réaction avec une base faible en milieu aqueux, par exemple les carbonates, phosphates, citrates de sodium, potassium ou ammonium ou leur mélanges, de préférence le carbonate de sodium à une température allant de l'ambiante, par exemple, 20° C jusqu'à environ 80° C, sous agitation et de préférence en augmentant l'agitation et progressivement la température depuis environ 45° C jusqu'à environ 75° C. On observe alors une séparation entre une phase aqueuse et une phase organique et l'on peut accentuer cette séparation, par exemple par addition d'une solution saturée de chlorure de sodium.

Il est possible par ce procédé de faire réagir seulement les acides gras, qui, sous forme de savons, deviennent hydrosolubles. La phase aqueuse obtenue peut être alors séparée facilement de la phase organique contenant les esters liposolubles après décantation, par exemple par centrifugation. Les acides gras libres peuvent être recueillis par acidification de la phase aqueuse par un acide, par exemple l'acide chlorhydrique concentré, extraits par un solvant, par exemple l'hexane et le solvant doivent être ensuite éliminé, par exemple par évaporation.

Les exemples ci-après illustrent l'invention. Dans ceux-ci, les pourcentages et parties sont pondéraux, sauf indication contraire. En ce qui concerne l'analyse quantitative des acides gras libres, celle-ci est effectuée, après méthylation par le chlorure d'acétyle, par chromatographie en phase gazeuse (CPG), donc sur la base des esters méthyliques.

### Exemple 1

### 1.1. Préparation du mélange d'acides gras de départ.

On utilise comme matière de départ un mélange d'acides gras provenant de l'hydrolyse enzymatique d'une huile de pépins de cassis au moyen d'une lipase non spécifique de Candida cylindracea. Le mélange d'acide gras est obtenu de la manière suivante:

On prépare une émulsion huile-dans-l'eau contenant 20 % d'huile de pépins de cassis et 1,2 % de lécithine de soja (Asol 100 (R), Lucas Meyer) mise en solution dans 78,8 % d'une solution aqueuse de tampon phosphate 0,025 M de pH 6,88 et en effectuant 5 passages dans un microfluidiseur (110T, Microfluidics Corporation, Newton), ce qui conduit à un diamètre moyen des gouttelettes d'huile de 450 nm environ.

On solubilise la lipase de Candida cylindracea, type B, Biocatalysts Ltd., Cardiff, Angleterre, dans le tampon phosphate, puis on la centrifuge à 4000 g pendant 20 min. pour éliminer les résidus insolubles. Le surnageant est utilisé pour les expériences. 10 ml de l'émulsion précédente (contenant 2 g d'huile de pépins de cassis) sont placés dans un Erlenmeyer bouché de 25 ml, dans un bain thermostaté à température 37° C sous agitation magnétique à 250 tour/min. à laquelle on ajoute la solution enzymatique correspondant à 0,2 g de lipase.

Après réaction pendant 4 h, on centrifuge le milieu à 4000 g pour casser l'émulsion et on récupère la phase lipidique par extraction à l'éther. On lave l'extrait à l'eau, on le sèche sur sulfate de sodium, puis on élimine le solvant par évaporation. On conserve les acides gras obtenus à -25° C à l'abri de la lumière et sous azote.

On récupère la lipase en solution dans la phase aqueuse ainsi que le glycérol formé par ultrafiltration (module YM10, seuil de coupure 10000, Amicon, Denver, USA), ce qui donne une solution concentrée de lipase qui peut être réutilisée.

Le taux d'hydrolyse, correspondant au pourcentage d'acides gras libérés au cours de la réaction correspond à 99,9 %, déterminé par titration acide-base à l'aide d'un titroprocesseur 692 de Metrohm. L'échantillon à analyser, dissout dans 25 ml d'un mélange équivolumique d'éthanol et d'éther éthylique a été titré par une solution alcoolique de KOH de concentration 0,1 N.

Le mélange d'acides gras a la composition suivante déterminée par CPG sous forme d'esters méthyliques:

| **Acides gras** | **%** |
|---|---|
| C 16:0 | 7,1 |
| C 18:0 | 1,7 |
| C 18:1 | 13,3 |
| C 18:2 | 45,6 |
| C 18:3 gamma | 15,5 |
| C 18:3 alpha | 12,2 |
| C 18:4 | 2,9 |
| C 20:1 | 0,8 |
| C 20:2 | 0,2 |
| Autres | 0,8 |

### 1.2. Estérification

On effectue l'estérification des acides gras par le méthanol en utilisant 900 mg de mélange d'acides gras dans un mélange de 11 ml d'hexane et de 1 ml de méthanol et 1200 mg d'enzyme immobilisée, Lipozyme TM 20 de Mucor Miehei, Novo Nordisk A/S, Danemark. Cette enzyme a une spécificité accrue pour les positions 1 et 3 du squelette du glycérol des triglycérides par rapport à la position 2. La réaction s'effectue à température ambiante dans un ballon en verre muni d'un barreau magnétique et placé sur un agitateur mécanique pendant 20 h. Après réaction, on filtre l'enzyme qui, après rinçage et réhydratation avec 10 % en volume d'eau, peut être réutilisée. On obtient un mélange d'acides gras libres et d'esters méthyliques d'acides gras.

### 1.3. Séparation des acides gras et des esters méthyliques

On chauffe 20 g du mélange d'acides gras et d'esters méthyliques d'acides gras précédent à 40° C, puis on ajoute 1,1 g de carbonate de sodium en solution dans de l'eau sous agitation. On augmente ensuite la vitesse d'agitation et on élève la température à 75° C. Lorsque cette dernière température est atteinte, on arrête le chauffage et on ajoute 20 ml d'eau salée saturée. On constate alors la formation d'une phase organique et d'une phase aqueuse. On centrifuge ensuite le tout pendant 10 min. à 3000 t/min., on sépare les deux phases, puis on acidifie la phase aqueuse qui contient les savons avec une solution d'acide chlorhydrique. On extrait ensuite les acides gras formés à l'hexane, puis on évapore l'hexane. On recueille ainsi 1,5 g d'acides gras enrichis en GLA et en SA ayant la composition suivante (CPG des esters méthyliques):

| **Acides gras** | **%** |
|---|---|
| C 16:0 | 1,3 |
| C 18:1 | 2 |
| C 18:2 | 6,5 |
| C 18:3 gamma | 73,4 |
| C 18:3 alpha | 2,4 |
| C 18:4 | 13,6 |
| Autres | 0,8 |

### Exemple 2

On procède comme à l'exemple 1, sauf que dans l'étape d'estérification (correpondant à 1.2 de l'exemple 1), on utilise un mélange de solvants constitué de 9 ml de méthanol et de 1 ml d'eau.

On obtient ainsi 64,6 % de GLA et 11,8 % de SA dans la phase enrichie (constituée par la partie non estérifiée obtenue à partir des savons).

### Exemples 3-7

On procède comme à l'exemple 1, mis à part le fait que, dans l'étape d'estérification (correpondant à 1.2 de l'exemple 1), on varie la durée de la réaction enzymatique comme indiqué et avec les résultats de % de GLA et de SA dans la phase enrichie indiqués ci-après (déterminé par CPG des esters méthyliques):

| **Exemple** | **3** | **4** | **5** | **6** | **7** |
|---|---|---|---|---|---|
| Temps de réaction, h | 5 | 10 | 30 | 40 | 70 |
| GLA % | 70 | 73,5 | 77,6 | 75,6 | 74,8 |
| SA % | 12 | 13 | 12,6 | 13,3 | 12,8 |

### Exemples 8-15

On procède comme à l'exemple 1 (paragraphe 1.2) à l'estérification d'un mélange d'acides gras d'huile de pépins de cassis avec des enzymes de différentes origines et régio-spécificités par réaction à la température ambiante pendant 20 h. On évalue les résultats obtenus en déterminant la composition en acides gras de la fraction estérifiée (par CPG sous forme d'esters méthyliques):

| **Exemple** | **8** | **9** | **10** | **11** | **12** | **13** | **14** | **15** |
|---|---|---|---|---|---|---|---|---|
| Enzyme de | Rhizopus javanicus, Biocatalysts | Penicillium cyclopium | Penicillium roqueforti | Geotrichum candidum | Lipomod, mélange de lipases | Candida cylindracea type OF | Rhizopus javanicus, type F-AP 15 | Aspergillus niger, type AP 6 |
| C 13:0 | - | - | - | 0,5 | - | - | - | - |
| C 16:0 | 7,6 | 5,8 | 8,8 | 11,2 | 8,4 | 7,9 | 8,1 | 7,6 |
| C 16:1 | - | 0,1 | - | - | - | - | - | - |
| C 18:0 | 1,2 | 1,1 | 1,8 | 1,8 | 1,5 | 1,4 | 1,3 | 1,4 |
| C 18:1 | 15,8 | 15,7 | 16,3 | 12,8 | 16,9 | 15,9 | 15,4 | 17,4 |
| C 18:2 | 57 | 59 | 54,9 | 48,7 | 56,8 | 56,2 | 56,1 | 57,7 |
| C 18:3 gamma | 2,1 | 1,6 | 2,2 | 1,2 | 1,8 | 2 | 5,1 | 2 |
| C 18:3 alpha | 14,5 | 15,3 | 13,5 | 7,2 | 12,3 | 14,4 | 12,9 | 13 |
| C 18:4 | - | - | - | 1,8 | 1 | 0,3 | - | - |
| C 20:1 | 0,6 | 0,6 | 0,8 | 0,6 | - | 0,8 | 0,6 | 0,8 |
| C 20:2 | - | 0,2 | - | - | - | 0,2 | - | - |
| Autres | 1,3 | 0,6 | 1,8 | 14,2 | 1,4 | 0,8 | 0,5 | 0,1 |
| -: Non quantifiable | | | | | | | | |

Les résultats précédents montrent que dans tous les cas la fraction estérifiée est apauvrie en GLA et en SA, ce qui indique que ces deux acides ne sont presque pas estérifiés et donc que la fraction des acides gras libres est enrichie en ces acides.

### Exemple 16

On utilise comme matière première un hydrolysat total d'une huile synthétique TGA de Suntory Ltd, Tokyo, Japon, extraite de fongus Mortierella, très riche en acide arachidonique. Après hydrolyse des triglycérides, on estérifie le mélange d'acides gras libres comme à l'exemple 1 (paragraphe 1.2) avec le Lipozyme TM 20 durant 20 h à la température ambiante. On sépare ensuite les esters méthyliques des acides gras libres par chromatographie sur couche mince, puis on analyse la composition du mélange d'acides gras libres. La composition du mélange d'acides gras de départ et celle du mélange d'acides gras libres obtenu après estérification (déterminées par CPG des esters méthyliques) sont indiquées ci-après:

| **Composition des acides gras %** | **De l'huile TGA** | **De la fraction estérifiée** |
|---|---|---|
| C 14:0 | 0,7 | - |
| C 16:0 | 17,2 | 5,6 |
| C 16:1 | 0,2 | - |
| C 17:0 | 0,3 | 0,2 |
| C 18:0 | 9,2 | 5,5 |
| C 18:1 | 22,3 | 7,7 |
| C 18:2 | 8,3 | 2,4 |
| C 18:3 gamma | 1,7 | 6,5 |
| C 18:3 alpha | 1 | - |
| C 20:0 | 0,9 | 0,9 |
| C 20:1 | 1 | 0,7 |
| C 20:2 | 0,7 | 0,5 |
| C 20:3 | 3,9 | 13,1 |
| C 20:4 | 20,2 | 40,2 |
| C 22:0 | 3,5 | 4,2 |
| C 22:6 | 8,2 | 11,2 |
| Autres | 0,7 | 1,5 |
| -: Non quantifiable | | |

Les résultats précédents montrent que, comme pour l'huile de pépins de cassis, ce sont surtout les acides gras tri-insaturés et plus qui sont enrichis dans la fraction acides gras libres. On remarque également que, malgré son faible pourcentage dans l'huile TGA, le GLA est enrichi sélectivement par rapport à l'acide alphalinolénique (ALA).

## Revendications

1. Procédé essentiellement enzymatique de fractionnement d'acides gras polyinsaturés, caractérisé par le fait que l'on conduit l'estérification d'un mélange d'acides gras par catalyse enzymatique avec le méthanol, puis que l'on sépare les esters méthyliques formés des acides gras n'ayant pas réagi par saponification des acides gras par une base faible en milieu aqueux, que l'on extrait les savons formés à l'eau, que l'on acidifie la phase aqueuse, que l'on extrait les acides gras formés par l'hexane et que l'on obtient une fraction enrichie en acides gras polyinsaturés désirés.

2. Procédé selon la revendication 1, caractérisé par le fait que l'on utilise un mélange d'acides gras de départ provenant de l'hydrolyse enzymatique totale des triglycérides d'une huile riche en acides gras polyinsaturés.

3. Procédé selon la revendication 2, caractérisé par le fait que l'huile mise en oeuvre est une huile riche en acides gras des séries n-3 et n-6, notamment en acide gammalinolénique, notamment choisie parmi l'huile de pépins de cassis, l'huile de bourrache et l'huile d'onagre.

4. Procédé selon la revendication 1, caractérisé par le fait que la saponification a lieu par réaction avec à titre de base faible un carbonate, phosphate, citrate de sodium, potassium ou ammonium ou leur mélanges et que l'on obtient une séparation du milieu réactionnel entre une phase aqueuse et une phase organique.

5. Procédé selon la revendication 4, caractérisé par le fait que la saponification a lieu par réaction avec une solution aqueuse de carbonate de sodium.

6. Procédé selon la revendication 4, caractérisé par le fait que la réaction a lieu à une température allant de 20 à 80° C sous agitation.

7. Procédé selon la revendication 4, caractérisé par le fait que la réaction a lieu en augmentant l'agitation et progressivement la température depuis environ 45° C jusqu'à environ 75° C.

8. Procédé selon la revendication 4, caractérisé par le fait que l'on accentue la séparation des phases par addition d'une solution saturée de chlorure de sodium.

9. Procédé selon la revendication 4, caractérisé par le fait que l'on sépare la phase aqueuse contenant les acides gras hydrosolubles de la phase organique contenant les esters liposolubles par centrifugation.

10. Procédé selon la revendication 4, caractérisé par le fait que l'on recueille les acides gras libres par acidification de la phase aqueuse par un acide, notamment l'acide chlorhydrique concentré, puis extraction par l'hexane et élimination de l'hexane, notamment par évaporation.

## Claims

1. Essentially enzymatic process for fractionating polyunsaturated fatty acids, characterized in that the esterification of a mixture of fatty acids is carried out by enzymatic catalysis with methanol, the methyl esters formed are then separated from the unreacted fatty acids by saponification of the fatty acids with a weak base in an aqueous medium, that the soaps formed are extracted with water, that the aqueous phase is acidified, that the fatty acids formed are extracted with hexane and that a fraction is obtained enriched in the required polyunsaturated fatty acids.

2. Process according to claim 1, characterized in that a starting mixture of fatty acids is used which is derived from the total enzymatic hydrolysis of the triglycerides of an oil rich in polyunsaturated fatty acids.

3. Process according to claim 2, characterized in that the oil used is an oil rich in fatty acids of the n-3 and n-6 series, in particular in gammalinolenic acid, selected in particular from blackcurrant seed oil, borage oil and evening primrose oil.

4. Process according to claim 1, characterized in that saponification takes place by reaction with, as a weak base, a carbonate, phosphate or citrate of sodium, potassium or ammonium or mixtures thereof and that separation of the reaction medium is obtained between an aqueous phase and an organic phase.

5. Process according to claim 4, characterized in that saponification takes place by reaction with an aqueous solution of sodium carbonate.

6. Process according to claim 4, characterized in that the reaction takes place at a temperature of 20 to 80°C with stirring.

7. Process according to claim 4, characterized in that the reaction is carried out while increasing stirring and raising the temperature progressively from approximately 45°C to approximately 75°C.

8. Process according to claim 4, characterized in that the separation of the phases is accentuated by adding a saturated solution of sodium chloride.

9. Process according to claim 4, characterized in that the aqueous phase containing water-soluble fatty acids is separated from the organic phase containing the liposoluble esters by centrifuging.

10. Process according to claim 4, characterized in that the free fatty acids are collected by acidifying the aqueous phase with an acid, in particular concentrated hydrochloric acid, followed by extraction with hexane and elimination of the hexane, in particular by evaporation.

## Patentansprüche

1. Im wesentlichen enzymatisches Verfahren zur Fraktionierung von mehrfach ungesättigten Fettsäuren, **dadurch gekennzeichnet**, daß man die Veresterung einer Mischung von Fettsäuren unter enzymatischer Katalyse mit Methanol durchführt, wonach man die gebildeten Methylester von den Fettsäuren, die nicht reagiert haben, durch Verseifung der Fettsäuren mit einer schwachen Base in einem wässrigen Medium abtrennt, daß man die gebildeten Seifen mit Wasser extrahiert, daß man die wässrige Phase ansäuert, daß man die gebildeten Fettsäuren mit Hexan extrahiert, und daß man eine an den gewünschten mehrfach ungesättigten Fettsäuren angereicherte Fraktion gewinnt.

2. Verfahren nach Anspruch 1, dadurch gekennzeichnet, daß man eine Ausgangs-Fettsäuremischung verwendet, die erhalten wurde durch vollständige enzymatische Hydrolyse von Triglyceriden eines Öls, das an mehrfach ungesättigten Fettsäuren reich ist.

3. Verfahren nach Anspruch 2, dadurch gekennzeichnet, daß das eingesetzte Öl ein Öl ist, das reich ist an Fettsäuren der Serien n-3 bis n-6, insbesondere an gamma-Linolensäure, und das insbesondere ausgewählt ist aus Johannisbeerkernöl, Borretschöl und Nachtkerzenöl.

4. Verfahren nach Anspruch 1, dadurch gekennzeichnet, daß man die Verseifung bewirkt durch Reaktion mit einem Carbonat, Phosphat, Citrat von Natrium, Kalium oder Ammonium oder deren Mischungen als schwache Base, und daß man eine Auftrennung des Reaktionsmilieus in eine wässrige Phase und eine organische Phase erhält.

5. Verfahren nach Anspruch 4, dadurch gekennzeichnet, daß man die Verseifung durch Reaktion mit einer wässrigen Lösung von Natriumcarbonat bewirkt.

6. Verfahren nach Anspruch 4, dadurch gekennzeichnet, daß man die Reaktion bei einer Temperatur von 20 bis 80°C unter Rühren durchführt.

7. Verfahren nach Anspruch 4, dadurch gekennzeichnet, daß man die Reaktion durchgeführt, indem man das Rühren verstärkt und die Temperatur von etwa 45°C auf etwa 75°C erhöht.

8. Verfahren nach Anspruch 4, dadurch gekennzeichnet, daß man die Phasentrennung durch Zugabe einer gesättigten Natriumchloridlösung verbessert.

9. Verfahren nach Anspruch 4, dadurch gekennzeichnet, daß man die die wasserlöslichen Fettsäuren enthaltende wässrige Phase von der die lipidlöslichen Ester enthaltenden organischen Phase durch Zentrifugieren abtrennt.

10. Verfahren nach Anspruch 4, dadurch gekennzeichnet, daß man die freien Fettsäuren durch Ansäuern der wässrigen Phase mit einer Säure, insbesondere konzentrierter Chlorwasserstoffsäure, gewinnt, gefolgt von ihrer Extraktion mit Hexan und der Entfernung des Hexans, insbesondere durch Verdampfen.
